# EUROPEAN PATENT SPECIFICATION

(11) **EP 3 019 164 B1**
(45) Date of publication and mention of the grant of the patent: **19.08.2020**
(21) Application number: 14823046.9
(22) Date of filing: 10.07.2014
(51) Int. Cl.: A61K 31/16, A61P 25/00, A61P 29/00

(54) **USE OF N-ACETYLCYSTEINE AMIDE IN THE TREATMENT OF PENETRATING HEAD INJURY**
VERWENDUNG VON N-ACETYLCYSTEINAMID IN DER BEHANDLUNG VON PENTRIERENDER KOPFLÄSION
EMPLOI DE N-ACÉTYLCYSTÉINE AMIDE DANS LE TRAITEMENT DE LÉSIONS PROFONDES À LA TÊTE

(30) Priority: 10.07.2013 US 201361844653 P; 26.07.2013 US 201361858871 P; 15.10.2013 US 201361891034 P
(43) Date of publication of application: 18.05.2016
(73) Proprietor: Goldstein, Glenn A., New York, NY 10022 (US)
(72) Inventor: GOLDSTEIN, Glenn A., New York, NY 10022 (US)
(74) Representative: Høiberg P/S
(86) International application number: PCT/US2014/046166
(87) International publication number: WO 2015/006569

(56) References cited:
- WO-A2-2006/116353
- WO-A2-2011/044230

## Description

### BACKGROUND

There is a need in the art for compounds and therapeutic aspects to treat pathogenesis associated with penetrating head injury. There is a specific needs for therapeutics to increase the viability of neuronal cells subject to penetrating head injury. The disclosure below provides such therapeutics.

### SUMMARY

The disclosure provides a method of treating penetrating head injury in a subject in need thereof comprising administering to the subject a composition comprising a therapeutically effective amount of N-acetylcysteine amide (NACA). In one embodiment of the present disclosure, the composition further comprises a pharmaceutically acceptable salt or excipient.

The treatment of the penetrating head injury comprises penetration of the dura mater or the brain caused by a gunshot.

In another embodiment of the present disclosure, the NACA is administered systemically. According to these embodiments of the present disclosure, the NACA is administered intraperitoneally, intravenously, orally or transdermally. In another embodiment of the present disclosure, the NACA is administered directly onto or into a wound caused by the penetrating head injury.

In certain embodiments of the present disclosure, the subject is a mammal. The mammal can be a human.

In other embodiments of the present disclosure, the NACA is administered at between 5 and 10,000; between 75 and 600; or between 200 and 400 mg/kg to the subject.

In certain embodiments of the present disclosure, the brain tissue comprises synaptic cells. According to these embodiments of the present disclosure, RCR and/or respiration rate is increased in the synaptic cells. In other embodiments of the present disclosure, the brain tissue comprises non-synaptic cells. According to these embodiments, RCR and/or respiration rate is increased in the non-synaptic cells. In other embodiments of the present disclosure, the non-synaptic cells comprise neuronal soma cells or glial cells.

### Brief Description of the Drawings

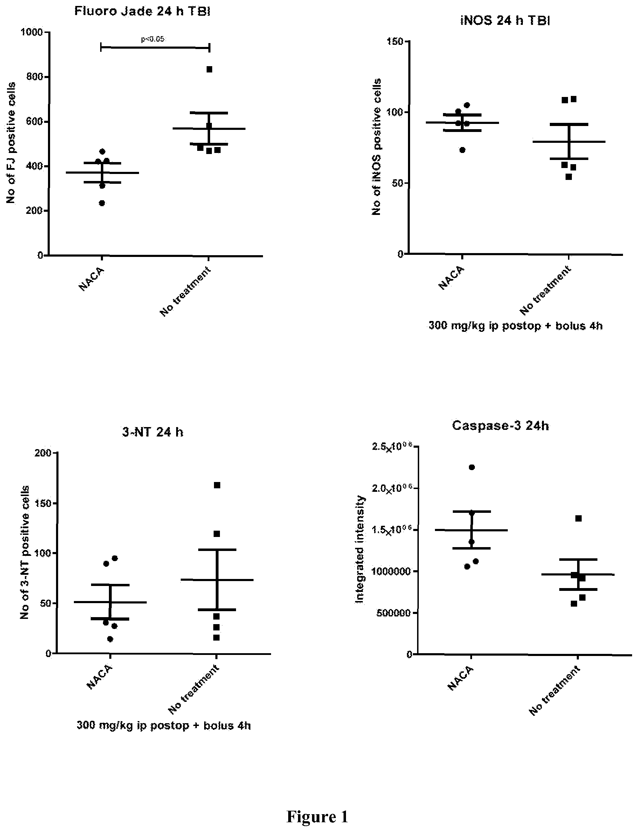

Figure 1 shows four graphs showing the relative occurrence or abundance of substances found using immunohistochemistry.

### DETAILED DESCRIPTION

The invention is defined by the appended claims. Any embodiment not falling under the scope of the appended claims does not form part of the invention.

The present disclosure provides the use of N-acetylcysteine amide (NAC amide or NACA) or a physiologically acceptable derivative, salt, or ester thereof, to treat disorders, conditions, pathologies and diseases that result from, or are associated with penetrating head injury. NACA are provided for use in methods and compositions for improving and treating penetrating head injury.

As used herein, a "subject" within the context of the present disclosure encompasses, without limitation, mammals, e.g., humans, domestic animals and livestock including cats, dogs, cattle and horses. A "subject in need thereof" is a subject having one or more manifestations of disorders, conditions, pathologies, and diseases as disclosed herein in which administration or introduction of NAC amide be considered beneficial by those of ordinary skill in the art.

"Therapeutic treatment" or "therapeutic effect" means any improvement in the condition of a subject treated by the methods of the present disclosure, including obtaining a preventative or prophylactic effect, or any alleviation of the severity of signs or symptoms of a disorder, condition, pathology, or disease or its sequelae, including those caused by other treatment methods (e.g., inflammation), which can be detected by means of physical examination, laboratory, or instrumental methods and considered statistically and/or clinically significant by those skilled in the art.

"Prophylactic treatment" or "prophylactic effect" means prevention of any worsening in the condition of a subject treated by the methods of the present disclosure, as well as prevention of any exacerbation of the severity of signs and symptoms of a disorder, condition, pathology, or disease or its sequelae, including those caused by other treatment methods (e.g., chemotherapy and radiation therapy), which can be detected by means of physical examination, laboratory, or instrumental methods and considered statistically and/or clinically significant by those skilled in the art.

In another aspect of the present disclosure, NAC amide is used in the treatment and/or prevention of disorders involving, hair, nails, and mucosal surfaces when applied topically. In accordance with the disclosure, compositions for topical administration are provided that include (a) NAC amide, or derivatives thereof, or a suitable salt or ester thereof, or a physiologically acceptable composition containing NAC amide or its derivatives; and (b) a topically acceptable vehicle or carrier.

Another aspect of the present disclosure provides a compound of the formula I: 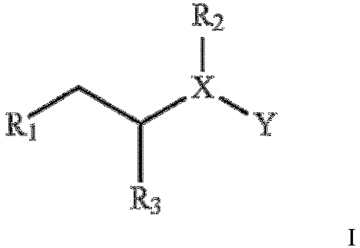 wherein: R₁ is OH, SH, or S--S--Z; X is C or N; Y is NH₂, OH, CH₃-C=O, or NH-CH₃; R.sub.2 is absent, H, or =O R₃ is absent or 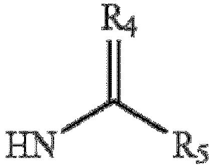 wherein: R₄ is NH or O; R₅ is CF₃, NH₂, or CH₃
and wherein: Z is 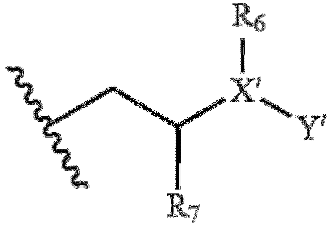 with the proviso that if R₁ is S--S--Z, X and X' are the same, Y and Y' are the same, R₂ and R₆ are the same, and R₃ and R₇ are the same.

The present disclosure also provides a NAC amide compound comprising the compounds disclosed herein. Other derivatives are disclosed in U.S. Patent No. 8,354,449.

### Penetrating Head Wound

According to certain embodiments of the present disclosure, NACA are used in the treatment of penetrating head wounds. In certain embodiments of the present disclosure, the injuries or wounds are caused by trauma or surgery. Penetrating head wounds include wounds that produce any degree of penetration into the head. The penetration can be any one or more layers of the head of a subject. These layers can include the skin, underlying muscle, connective tissue, bone, ear, ear canal, eye, eye socket, mouth, teeth, jaw, skull and brain. Parts of the brain that can be penetrated according to the penetrating head wounds described herein include the cerebellum, medulla, pons, hypothalamus, thalamus, optic tectum, neocortex, basal ganglia, olfactory bulb and cerebral cortex.

In certain embodiments of the present disclosure, administration of NACA reduces symptoms associated pathology associated with the penetrating head wound. The symptoms associated pathology can present as symptoms including subdural hematoma, epidural hematoma, subarachnoid hematoma, increase in intracranial pressure, ischemia and shock. In certain embodiments of the present disclosure, administration of NACA leads to the alleviation and/or improvement of one or more of these symptoms.

In certain embodiments of the present disclosure, administration of NACA is useful for treating secondary pathologies associated with the primary penetrating head injury. These injuries can include a secondary traumatic brain injury of any kind. The secondary traumatic brain injury can include concussion, contusion or laceration. The secondary traumatic brain injury can be of any severity. The Secondary traumatic brain injury can be mild, moderate or severe. The Secondary traumatic brain injury can be accompanied by cerebral hemorrhage. Cerebral hemorrhage includes epidural hematoma, subdural hematoma, subarachnoid hemorrhage, and intraventricular hemorrhage. Symptoms of the secondary traumatic brain injury include headache, vomiting, nausea, lack of motor coordination, dizziness, difficulty balancing, lightheadedness, blurred vision or tired eyes, ringing in the ears, bad taste in the mouth, fatigue or lethargy, changes in sleep patterns, convulsions, an inability to awaken, dilation of one or both pupils, slurred speech, aphasia, dysarthria, weakness or numbness in the limbs, loss of coordination, confusion, restlessness, agitation, changes in appropriate social behavior, deficits in social judgment, cognitive changes, problems with sustained attention, processing speed, and executive functioning and alexithymia. NACA can be used to treat any of these types of traumatic brain injuries and to improve any of the symptoms associated with traumatic brain injury.

According to certain embodiments of the present disclosure, NACA or derivatives thereof can be administered systemically or on, in or near the site of the wound caused by the penetrating head injury. Systemic administration methods include intraperitoneal, intravenous, oral or transdermal administration. Administration onto the site of the wound can include injection or transdermal delivery.

Doses, amounts or quantities of NACA, or derivatives thereof, are determined on an individual basis. As is appreciated by the skilled practitioner in the art, dosing is dependent on the severity and responsiveness of the penetrating head injury to be treated, but will normally be one or more doses per day, with course of treatment lasting from several days to several months, or until a cure is effected or a diminution of disease state is achieved. Persons ordinarily skilled in the art can easily determine optimum dosages, dosing methodologies and repetition rates. For example, a pharmaceutical formulation for orally administrable dosage form can comprise NACA, or a pharmaceutically acceptable salt, ester, or derivative thereof in an amount equivalent to at least 25-500 mg per dose, or in an amount equivalent to at least 50-350 mg per dose, or in an amount equivalent to at least 50-150 mg per dose, or in an amount equivalent to at least 25-250 mg per dose, or in an amount equivalent to at least 50 mg per dose. NAC amide or a derivative thereof can be administered to both human and non-human mammals. It therefore has application in both human and veterinary medicine. In certain embodiments of the present disclosure, treatment with NACA can last for 1, 2, 3, 4, 5, 10, 15, 20, 25, 30, 35, 40, 45 or 50 weeks.

### Pharmaceutical Compositions

As used herein the term "pharmaceutical composition" refers to a preparation of one or more of the components described herein, or physiologically acceptable salts or prodrugs thereof, with other chemical components such as physiologically suitable carriers and excipients. The purpose of a pharmaceutical composition is to facilitate administration of a compound to an organism. The term "prodrug" refers a precursor compound that can hydrolyze, oxidize, or otherwise react under biological conditions (in vitro or in vivo) to provide the active compound. Examples of prodrugs include, but are not limited to, metabolites of NSAIDs that include biohydrolyzable moieties such as biohydrolyzable ainides, biohydrolyzable esters, biohydrolyzable carbamates, biohydrolyzable carbonates, biohydrolyzable ureides, and biohydrolyzable phosphate analogues.

The term "excipient" refers to an inert or inactive substance added to a pharmaceutical composition to further facilitate administration of a compound. Non-limiting examples of excipients include calcium carbonate, calcium phosphate, various sugars and types of starch, cellulose derivatives, gelatin, vegetable oils and polyethylene glycols.

The pharmaceutical compositions of the present disclosure comprise NAC Amide and may also include one or more additive drugs (e.g., additional active ingredients), such as, but not limited to, NSAIDs, antibiotics, conventional anti-cancer and/or anti-inflammatory agents that may be suitable for combination therapy.

The pharmaceutical compositions of the present disclosure may be manufactured by processes well known in the art, e.g., by means of conventional mixing, dissolving, granulating, grinding, pulverizing, dragee-making, levigating, emulsifying, encapsulating, entrapping or by lyophilizing processes.

The compositions for use in accordance with the present disclosure thus may be formulated in conventional manner using one or more pharmaceutically acceptable carriers comprising excipients and auxiliaries, which facilitate processing of the active compounds into preparations which can be used pharmaceutically. Proper formulation is dependent upon the route of administration chosen.

The term "administration" or any lingual variation thereof as used herein is meant any way of administration. The one or more of NAC Amide and at least one additional drug may be administered in one therapeutic dosage form or in two separate therapeutic dosages such as in separate capsules, tablets or injections. In the case of the two separate therapeutic dosages, the administration may be such that the periods between the administrations vary or are determined by the practitioner. It is however preferred that the second drug is administered within the therapeutic response time of the first drug. The one or more of NAC Amide and at least one additional drug which may be administered either at the same time, or separately, or sequentially, according to the disclosure, do not represent a mere aggregate of known agents, but a new combination with the valuable property that the effectiveness of the treatment is achieved at a much lower dosage of said at least one additional drug.

The pharmaceutical compositions of the present disclosure may be administered by any convenient route, for example, by infusion or bolus injection, by absorption through epithelial or mucocutaneous linings (e.g., oral mucosa, rectal and intestinal mucosa, etc.) and may be administered together with any other therapeutic agent. Administration can be systemic or local.

Various delivery systems are known, e.g., encapsulation in liposomes, microparticles, microcapsules or capsules, that may be used to administer the compositions of the disclosure. Methods of administration include but are not limited to intradermal, intramuscular, intraperitoneal, intravenous, subcutaneous, epidural, oral, sublingual, intranasal, intracerebral, intravaginal, transdermal, rectally (including by suppository or enema), by inhalation, or topically to the cars, nose, eyes, or skin. The preferred mode of administration is left to the discretion of the practitioner, and will depend in part upon the site of the medical condition (such as the site of cancer) and the severity of thereof.

For example, for injection the composition of the disclosure may be formulated in aqueous solutions, preferably in physiologically compatible buffers such as Hank's solution, Ringer's solution, or physiological saline buffer. For transmucosal administration, penetrants appropriate to the barrier to be permeated are used in the formulation. Such penetrants for example DMSO, or polyethylene glycol are generally known in the art.

For oral administration, the composition can be formulated readily by combining the active components with any pharmaceutically acceptable carriers known in the art. Such "carriers" may facilitate the manufacture of such as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum, methyl cellulose, hydroxypropylmethyl-cellulose, sodium carbomethylcellulose, and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active components may be dissolved or suspended in suitable liquids, such as fatty oils, liquid paraffin, or liquid polyethylene glycols.

Dyestuffs or pigments may be added to the tablets or dragee coatings for identification or to characterize different combinations of active NSAID doses. In addition, stabilizers may be added.

Pharmaceutical compositions for parenteral administration include aqueous solutions of the active preparation in a water-soluble form. Additionally, suspensions of the active preparation may be prepared as oily injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl, cellulose, sorbitol or dextran. Optionally, the suspension may also contain suitable stabilizers or agents, which increase the solubility of the compounds, to allow for the preparation of highly concentrated solutions.

Alternatively, the composition may be in a powder form for constitution before use with a suitable vehicle, e.g., sterile, pyrogen-free water. The exact formulation, route of administration and dosage may be chosen by the physician familiar with the patient's condition. (See for example Fingl, et al., 1975, in "The Pharmacological Basis of Therapeutics", Chapter I, p. 1). Depending on the severity and responsiveness of the condition treated, dosing can also be a single administration of a slow release composition, with course of treatment lasting from several days to several weeks or until cure is effected or diminution of the disease state is achieved.

### EXAMPLES

### Example 1: NACA Reduces Neural Degeneration in Rats with Penetrating Head Injury.

A penetrating brain injury (non-fatal) was produced in rats. A needle was used to create a penetrating brain injury in rats. The rats were studied 24 hours post trauma. 5 rats received NACA (300 mg ip per kg body weight and a bolus after 4 hours) and were compared to 5 controls (i.e. trauma, no NACA). Significant increased survival of cortical neurons was shown in the rats administered NACA than controls. This was verified by Fluoro Jade staining of histological samples. Fluoro Jade stains degenerating neurons, giving the number of dying neurons. Among the NACA-treated group the number of degenerating cells ranged between 200 and 450, as compared to the untreated group of between 450 and 800.

### Example 2: Characterization of Neural Tissue in Rats with Penetrating Head Injury Administered NACA.

Penetrating head injury is the next result of a primary injury, in this case the needle penetration and a secondary injury, which involves cytokines and inflammation. The presence and relative amounts of certain inflammatory cytokines and other signaling molecules involved with inflammation will be measured to investigate the mechanism of the protective effect of NACA. These include p38 mitogen-activated protein kinase (MAPK) and inducible nitric oxide synthase (iNOS) signaling, as well as Bcl-2, Bax, and NF-KB expression.

### Example 3. Immunohistochemical Analysis of Brain Tissue of Rats with Penetrating Head Injury when Administered NACA

Focal traumatic brain injuries (TBIs) using a penetrating brain injury model were produced in male rats (n=5). After 2h and 24h the brains were removed, cut in 14 µm coronal sections and subjected to immunohistochemical analyses.

### Results

The level of fluoro jade stained neuronal degeneration was lower in the group treated with NACA after 24h. iNOS was upregulated in the perilesional area, not differing between groups. Oxidative stress detected by peroxynitrite surrogate marker 3-NT was detected in the perilesional area, not differing between groups.

## Claims

1. A composition comprising a therapeutically effective amount of N-acetylcysteine amide (NACA) for use in a method of treating penetrating head injury in a subject in need thereof, wherein the penetrating head injury comprises penetration of the dura mater or the brain, and wherein the penetration is caused by a gunshot.

2. The composition for the use of claim 1, wherein the composition further comprises a pharmaceutically acceptable salt or excipient.

3. The composition for the use of claim 1, wherein the NACA is administered systemically such as intraperitoneally, intravenously, orally or transdermally.

4. The composition for the use of claim 1, wherein the NACA is administered directly onto or into a wound caused by the penetrating head injury.

5. The composition for the use of claim 1, wherein the subject is a mammal such as a human.

6. The composition for the use of claim 1, wherein the NACA is administered at between 5 and 10,000 mg/kg, or at between 75 and 600 mg/kg, or at between 200 and 400 mg/kg to the subject.

7. The composition for the use of claim 1, wherein the brain tissue comprises synaptic cells.

8. The composition for the use of claim 7, wherein RCR and/or respiration rate is increased in the synaptic cells.

9. The composition for the use of claim 1, wherein the brain tissue comprises non-synaptic cells.

10. The composition for the use of claim 9, wherein RCR and/or respiration rate is increased in the non-synaptic cells.

11. The composition for the use of claim 9, wherein the non-synaptic cells comprise neuronal soma cells.

12. The composition for the use of claim 9, wherein the non-synaptic cells comprise glial cells.

## Patentansprüche

1. Zusammensetzung, umfassend eine therapeutisch wirksame Menge N-Acetylcysteinamid (NACA) zur Verwendung in einem Verfahren zum Behandeln einer penetrierenden Kopfverletzung bei einem Subjekt, das dessen bedarf, wobei die penetrierende Kopfverletzung eine Penetration der Dura mater oder des Hirns umfasst, und wobei die Penetration durch einen Schuss verursacht wird.

2. Zusammensetzung zur Verwendung nach Anspruch 1, wobei die Zusammensetzung ferner ein pharmazeutisch verträgliches Salz oder einen pharmazeutisch verträglichen Exzipienten umfasst.

3. Zusammensetzung zur Verwendung nach Anspruch 1, wobei das NACA systemisch, wie intraperitoneal, intravenös, oral oder transdermal verabreicht wird.

4. Zusammensetzung zur Verwendung nach Anspruch 1, wobei das NACA direkt auf oder in eine Wunde verabreicht wird, die durch die penetrierende Kopfverletzung verursacht wird.

5. Zusammensetzung zur Verwendung nach Anspruch 1, wobei das Subjekt ein Säuger wie ein Mensch ist.

6. Zusammensetzung zur Verwendung nach Anspruch 1, wobei das NACA zwischen 5 und 10.000 mg/kg oder zwischen 75 und 600 mg/kg oder zwischen 200 und 400 mg/kg an das Subjekt verabreicht wird.

7. Zusammensetzung zur Verwendung nach Anspruch 1, wobei das Hirngewebe synaptischen Zellen umfasst.

8. Zusammensetzung zur Verwendung nach Anspruch 7, wobei RCR und/oder Atmungsrate in den synaptischen Zellen erhöht sind/ist.

9. Zusammensetzung zur Verwendung nach Anspruch 1, wobei das Hirngewebe nicht-synaptische Zellen umfasst.

10. Zusammensetzung zur Verwendung nach Anspruch 9, wobei RCR und/oder Atmungsrate in den nicht-synaptischen Zellen erhöht ist.

11. Zusammensetzung zur Verwendung nach Anspruch 9, wobei die nicht-synaptischen Zellen neuronale Somazellen umfassen.

12. Zusammensetzung zur Verwendung nach Anspruch 9, wobei die nicht-synaptischen Zellen Gliazellen umfassen.

## Revendications

1. Composition comprenant une quantité thérapeutiquement efficace de N-acétylcystéine amide (NACA) pour une utilisation dans un procédé de traitement d'une lésion profonde à la tête chez un sujet en ayant besoin, dans laquelle la lésion profonde à la tête comprend la pénétration de la dure-mère ou du cerveau, et dans laquelle la pénétration est provoquée par un coup de feu.

2. Composition pour l'utilisation selon la revendication 1, dans laquelle la composition comprend en outre un sel ou excipient pharmaceutiquement acceptable.

3. Composition pour l'utilisation selon la revendication 1, dans laquelle le NACA est administré par voie systémique, comme par voie intrapéritonéale, intraveineuse, orale ou transdermique.

4. Composition pour l'utilisation selon la revendication 1, dans laquelle le NACA est administré directement sur ou dans une plaie causée par la lésion profonde à la tête.

5. Composition pour l'utilisation selon la revendication 1, dans laquelle le sujet est un mammifère, tel qu'un humain.

6. Composition pour l'utilisation selon la revendication 1, dans laquelle le NACA est administré entre 5 et 10 000 mg/kg, ou entre 75 et 600 mg/kg, ou entre 200 et 400 mg/kg au sujet.

7. Composition pour l'utilisation selon la revendication 1, dans laquelle le tissu cérébral comprend des cellules synaptiques.

8. Composition pour l'utilisation selon la revendication 7, dans laquelle le RCR et/ou la fréquence respiratoire sont augmentés dans les cellules synaptiques.

9. Composition pour l'utilisation selon la revendication 1, dans laquelle le tissu cérébral comprend des cellules non synaptiques.

10. Composition pour l'utilisation selon la revendication 9, dans laquelle le RCR et/ou la fréquence respiratoire sont augmentés dans les cellules non synaptiques.

11. Composition pour l'utilisation selon la revendication 9, dans laquelle les cellules non synaptiques comprennent des cellules de soma neuronal.

12. Composition pour l'utilisation selon la revendication 9, dans laquelle les cellules non synaptiques comprennent des cellules gliales.
